# EUROPÄISCHE PATENTSCHRIFT

(11) **EP 1 163 921 B1**
(45) Veröffentlichungstag und Bekanntmachung des Hinweises auf die Patenterteilung: **02.11.2006**
(21) Anmeldenummer: 01113576.1
(22) Anmeldetag: 13.06.2001
(51) Int. Cl.: A61M 15/00

(54) **Inhalationsvorrichtung**
Inhalator
Inhalateur

(30) Priorität: 14.06.2000 DE 10029119
(43) Veröffentlichungstag der Anmeldung: 19.12.2001
(73) Patentinhaber: Activaero GmbH, 35285 Gemünden (DE)
(72) Erfinder: Scheuch, Gerhard, 35285 Gemünden (DE); Sommerer, Knut, 81241 München (DE); Haas, Friedel, 82131 Gauting (DE); Müllinger, Bernhard, 84339 Unterdietfurt (DE); Roeder, Sascha, 85053 Ingolstadt (DE)
(74) Vertreter: Vossius & Partner

(56) Entgegenhaltungen:
- EP-A- 0 965 355
- EP-A- 1 036 569
- EP-A- 1 038 544
- US-A- 5 655 520
- US-A- 5 842 467

## Beschreibung

Die Erfindung betrifft eine Vorrichtung zum Inhalieren von dosierten Arzneistoffen in Aerosolform in die Lunge. Geeignete Arzneistoffe umfassen Analgetika, Antianginamittel, Antiallergica, Antihistamine und entzündungshemmende Mittel, Expectorantia, Antitussiva, Bronchodilatoren, Diuretika, Anticholingerica, Corticoide, Xanthine, Antitumormittel sowie therapeutisch wirksame Proteine oder Peptide wie Insulin oder Interferon.

Bevorzugt ist die Verabreichung von Arzneistoffen zur Behandlung von respiratorischen Erkrankungen wie Asthma, sowie Mittel zur Prophylaxe und Behandlung der Schleimhäute des Tracheo-Bronchialtraktes. Besonders bevorzugt ist die Verabreichung von Vitamin A.

Der Begriff Vitamin A steht für eine Reihe chemisch ähnlicher Verbindungen mit unterschiedlicher Wirkung im menschlichen und tierischen Organismus. Das Vitamin A ist für den Menschen essentiell, d.h. es entsteht ein Vitaminmangel, wenn es nicht mit der Nahrung zugeführt wird. Der Vitamin-A-Mangel äußert sich in verschiedenen Veränderungen im Bereich von Haut, Schleimhäuten und Augen. Symptome können beispielsweise Verhornung der Schleimhäute der Atemwege oder der Bindehaut des Auges, erhöhte Infektanfälligkeit sowie bei ausgeprägtem Mangel die Erblindung sein. Bei Zufuhr des Vitamins A sind die meisten mangelbedingten Veränderungen vor allem im Bereich der Schleimhäute rückbildungsfähig. Jedoch müssen bei systemischer Anwendung zur Erreichung einer Rückbildung, beispielsweise einer Plattenepithelmetaplasie, oder zur Verhinderung eines erneuten Auftretens solcher Veränderungen hohe Konzentrationen eingesetzt werden, die teilweise zu erheblichen Nebenwirkungen (Hirndrucksymptomatik, Leberzellstoffwechselstörungen, u.a.) führen. Außerdem verbietet sich der Einsatz hochdosierter Präparate in der Schwangerschaft aufgrund des Risikos fetaler Mißbildungen (Bauernfeind J. C.; The Safe Use of Vitamin A, The Nutrition Foundation, Washington D.C. 1980).

Des weiteren ist eine Zufuhr von Vitamin A auch in physiologischer Konzentration bei ernährungsbedingtem Proteinmangel und bei Störungen des Leberzellstoffwechsels, wie z.B. Entzündung oder Zirrhose, nicht zulässig, da durch die gleichzeitig gestörte Proteinsynthese (mangelnde Bildung des Transportproteins) der Leber eine Ausschleusung des Vitamins aus Speichern, in die es nach Resorption überführt wird, nicht möglich ist.

Hinzu kommt, daß durch die peripheren Zielgewebe, wie z.B. das Respirationsepithel, das Vitamin nur dann nach systemischer Gabe aufgenommen und seiner Funktion zugeführt werden kann, wenn es an eben dieses Transportprotein gebunden ist.

In der EP-A-0 352 412 wird zur Lösung dieser Problematik die Verwendung einer Zubereitung von Estern des Retinols sowie der Retinsäure zur inhalativen Applikation beschrieben. Hierdurch wird insbesondere eine topische Einwirkung des Wirkstoffs auf die Schleimhäute des Tracheo-Bronchialtraktes von Mensch und Tier ermöglicht. Damit wird die Vorbeugung und Behandlung von bestimmten Erkrankungen und Funktionsanomalien erleichtert, beispielsweise bestimmter zellulärer Differenzierungsstörungen, Plattenepithel-Metaplasien, neo-plastischen Veränderungen, eingeschränkter Aktivität des Flimmerepithels und Dysfunktion schleimbildender Zellen. Weiterhin kann diese Zubereitung für die Therapie oder als Adjuvans bei der Therapie von u.a. Bronchialkarzinomen, akuten und chronischen Bronchitiden und der bronchiopulmonalen Dysplasie von Neugeborenen eingesetzt werden. Aus klinischen Studien geht jedoch hervor, daß bei der inhalativen Applikation von Vitamin A unter Verwendung von üblichen Inhalationsvorrichtungen der Wirkstoff nur in unzureichenden Mengen an das Zielgewebe, das Flimmerepithel der Bronchialschleimhaut, verabreicht werden kann.

Die EP-A-1 038 544 offenbart eine Vorrichtung zur Flussbegrenzung bei niedrigen Differenzdrücken, insbesondere zur Begrenzung des Inhalationsvolumenstroms bei einer Inhalation von therapeutischen Aerosolen, die aus einem Gehäuse mit einer Einsaugöffnung, einer Aussaugöffnung und einem zwischen diesem angeordneten Strömungskanal mit einem flachen länglichen Querschnitt mit flexiblen großflächigen Wänden besteht. Der Querschnitt des Strömungskanals ist abhängig von dem Differenzdruck zwischen Aussaugöffnung und Einsaugöffnung sowie der Materialflexibilität des Wandmaterials zu einer Größe für einen vorgegebenen maximalen Inhalationsvolumenstrom verringerbar. Insbesondere Fig. 9 dieser Veröffentlichung zeigt einen Strömungsbereich zwischen Einsaugöffnung und Ansaugöffnungen mit sternförmigen bzw. radialen Stegen, die abwechselnd unterschiedliche Steglängen aufweisen.

Die Verabreichung von Arzneistoffen in Aerosolform durch Inhalation in die Lunge ist im wesentlichen durch vier Faktoren beeinflußt: (i) die Partikelgröße und die Partikeleigenschaften des Aerosol; (ii) das Atemzugvolumen des Patienten; (iii) der Atemfluß des Patienten; und (iv) die Morphometrie und Atemwege des Patienten. Während bisherige Systeme zwar Aerosole in geeigneten Teilchengrößenbereichen produzieren, werden bei bekannten Systemen die Parameter "Atemzugvolumen" und "Atemfluß" (Atemgeschwindigkeit) nur ungenügend oder gar nicht berücksichtigt. Dies führt zu einer unkontrollierten Inhalation des Aerosols, was wiederum dazu führt, daß eine unzureichende Menge an Aerosolteilchen die Lunge erreicht bzw. innerhalb der Lunge die zu therapierenden Bereiche (beispielsweise Alveolarbereich) nicht erreicht.

In der EP-A-0 965 355 wird eine Vorrichtung zum gesteuerten inhalatorischen Einbringen von Dosiermedikamenten in die Lunge vorgeschlagen. Dieser gesteuerte Inhalator weist einen geschlossenen Behälter auf, der mit einem vorbestimmbaren Aerosolvolumen befüllbar ist und aus dem das Aerosol mittels einer Steuereinrichtung für den Inhalationsfluss entnehmbar ist. Bei diesem bekannten Inhalator ist diese Steuereinrichtung entweder ein Verstellventil oder eine kritische Düse. Durch die Verwendung eines verstellbaren Ventils oder einer kritischen Düse kann eine Atemflusslimitierung erreicht werden.

In der EP-B-0 050 654 wird ein Atemgerät zur medikamentösen Behandlung der Lunge vorgeschlagen. Dieses Atemgerät weist eine aufblasbare Hülle auf, aus der mittels eines Mundstückes Aerosol inhaliert werden kann. Dieses Aerosol wir vor der Inhalation aus einer Kartusche über einen Zerstäuber in die aufblasbare Hülle eingebracht. Zur Begrenzung der Durchflussmenge der Luft durch das Mundstück bei der Inhalation weist das Mundstück eine Verengung auf. Diese Verengung limitiert den Atemfluß bei der Inhalation.

Die beiden genannten Inhalationsvorrichtungen zeichnen sich dadurch aus, daß eine Fluss-Limitierung erfolgt, d. h. während der Inspirationsphase steigt der Atemfluß lediglich langsam an und der Anstieg des Atemflusses nimmt stetig ab, was bei graphischer Darstellung des Atemflusses gegenüber der Zeit zu einer ständigen Abflachung der Kurve führt. Diese Flußlimitierung führt dazu, daß je nach Saugvermögen des Patienten der Atemfluß unterschiedlich ansteigt (und abflacht) und im schlimmsten Fall für die notwendige Behandlung nicht ausreichend ist. Dies bedeutet, daß bei den bekannten Inhalatoren die vorgesehene Flußlimitierung zu einer unzureichenden Aerosoldeposition führen kann.

US-A-5 655 520 betrifft ein flexibles Ventil zum Verabreichen einer konstanten Flußrate eines Medikaments von einem Vernebler. Hierzu ist eine Düse mit flexiblen Wänden vorgesehen, die eine Öffnung bilden, deren Querschnitt sich abhängig von dem Einatemvorgang des Patienten verändert, um eine konstante Flußrate bereitzustellen.

Demgegenüber liegt der Erfindung die Aufgabe zugrunde, eine Inhalationsvorrichtung bereitzustellen, die unabhängig von Patientencharakteristika den für die Inhalation von Aerosolen, insbesondere Vitamin A, notwendigen Atemfluß bereitstellt. Diese Aufgabe wird durch eine Inhalationsvorrichtung mit den Merkmalen der Ansprüche gelöst.

Die Erfindung geht von dem Grundgedanken aus, eine Steuereinrichtung bereitzustellen, die den Inhalationsfluss während der gesamten Inhalation des Aerosols im wesentlichen konstant hält. Dies bedeutet, daß erfindungsgemäß der Inhalationsfluss unmittelbar bei Beginn der Inspirationsphase auf seinen Maximalwert ansteigt, der für die ausreichende Verabreichung des Aerosols notwendig ist und auf diesem Maximalwert bleibt, solange vom Patienten während der Inhalation ein Mindestdruck erzeugt wird. Dieser Mindestdruck beträgt vorzugsweise höchstens 10 mbar und liegt bevorzugt im Bereich zwischen 5 und 10 mbar. Somit wird erfindungsgemäß bereits bei niedrigen Differenzdrücken eine Flußbegrenzung bereitgestellt.

Die erfindungsgemäße Inhalationsvorrichtung ist eine Kombination aus einem selbstexpandierenden Behältnis für ein vorbestimmtes Aerosolvolumen, einer Einrichtung zum Einbringen von Aerosol aus einem Aerosolspender in das Behältnis und einer Einrichtung zum Steuern des Inhalationsflusses, wobei die Steuereinrichtung den Inhalationsfluss während der gesamten Inhalation im wesentlichen konstant hält.

Erfindungsgemäß weist die Steuereinrichtung einen Strömungskanal mit einer Einlaßöffnung und einer Auslaßöffnung auf, die voneinander beabstandet an den beiden Enden des Strömungskanals angeordnet sind.

Der Strömungskanal wird beispielsweise durch eine großflächige, flexible Wand und eine parallel dazu angeordnete, im wesentlichen starre Wand gebildet. Die flexible Wand ist an der dem Strömungskanal abgewandten Seite durch eine Abdeckung abgedeckt. Die Auslaßöffnung des Strömungskanals mündet vorzugsweise in das Innere eines Gehäuses, das das Aerosolbehältnis umgibt. Vor der Inhalation des Aerosols wird dieses beispielsweise aus einer Kartusche in das Innere des Behältnisses eingebracht, vorzugsweise über eine Düse, wie etwa einen Vernebler. Dabei expandiert das Behältnis bis sich im voll expandierten Zustand ein durch das Behältnisvolumen vorbestimmtes Aerosolvolumen im Inneren des Behältnisses befindet. Sobald ein Patient über ein vorzugsweise vorgesehenes Mundstück das Aerosol aus dem Behältnis ansaugt, zieht sich dieses aufgrund des Soges zusammen. Der daraufhin im Gehäuseinneren entstehende Unterdruck wird über den Strömungskanal ausgeglichen. Der anliegende Unterdruck bewirkt dabei, daß sich die flexible Wand abhängig von der Größe des Unterdrucks zum Inneren des Strömungskanals hin wölbt, und dadurch dessen Querschnitt verringert. Diese Querschnittsverringerung bewirkt eine Begrenzung des Luftstromes durch den Strömungskanal in das Gehäuseinnere zum Druckausgleich, was wiederum den Aerosolfluss heraus aus dem Behältnis begrenzt. Durch die erfindungsgemäße Steuereinrichtung setzt bereits bei Drücken von 5 mbar eine selbsttätige Volumenstromregelung im Strömungskanal und somit selbsttätige Atemflussregelung ein. Der beim Einatmen des Aerosols entstehende Unterdruck bewirkt unmittelbar eine Querschnittsreduzierung des Strömungskanals aufgrund der flexiblen Wand, und zwar eine Reduzierung unmittelbar auf einen Grenzwert. Dies bewirkt, daß der Atemflussgrenzwert bereits unmittelbar bei Beginn der Inhalation erreicht wird und dieser bei durch das Ansaugen von der Lunge üblicherweise hervorgerufenen Drücken von 80 bis 100 mbar während der gesamten Inhalation beibehalten wird.

Die im wesentlichen starre Wand des Strömungskanals weist beispielsweise eine oder mehrere längliche, sich in Kanalrichtung erstreckende Vertiefungen bzw. Nuten auf, die voneinander durch entsprechende Stege beabstandet sind. Die flexible Wand, die vorzugsweise aus einem bioverträglichen Material wie etwa Silikon oder Kautschuk besteht, wölbt sich während der Inhalation in die Vertiefungen und liegt auf den Stegen auf. Die Stege verhindern ein vollständiges Verschließen des Strömungskanals und Begrenzen die Verringerung des Kanalquerschnitts.

Die erfindungsgemäße Inhalationsvorrichtung weist neben dem selbstexpandierbaren Behältnis und der Einbringungseinrichtung für das Aerosol eine Steuereinrichtung auf, bei der sternförmige Strömungskanäle zwischen vier sich sternförmig erstreckenden Stegen ausgebildet sind. Auf diesen Stegen liegt eine kreisförmige flexible Matte auf, die sich wie bei der ersten Ausführungsform bei Entstehen eines Unterdrucks in den Strömungskanälen in die Strömungskanäle wölbt, den Querschnitt reduziert und dadurch den Volumenstrom auf einem im wesentlichen konstanten Wert reguliert. Mindestens ein Steg weist eine größere Länge auf.

Durch die Einrichtung zum Einbringen von Aerosol aus einem Aerosolspender in das Behältnis wird verhindert, das ein Arzneistoff wie etwa Vitamin A in Form eines Aerosols direkt aus dem Aerosolspender in den Mundraum ausgegeben und inhaliert wird. Vielmehr wird der Patient angehalten, das Aerosol aus dem Aerosolspender in das Behältnis einzubringen, um dann das durch den Behälter definierte vorbestimmte Aerosolvolumen mittels der erfindungsgemäßen Inhalationsvorrichtung zu inhalieren. Vorzugsweise wird der Aerosolspender wie etwa eine Kartusche über einen Kragen mit einer Düse verbunden und an der Inhalationsvorrichtung gehalten. Über die Düse wird das Aerosol in das Innere des Behältnisses eingebracht.

Weitere bevorzugte Merkmale sind in den abhängigen Patentansprüchen angegeben.

Die erfindungsgemäße Inhalationsvorrichtung weist zahlreiche Vorteile auf. Die erfindungsgemäße Inhalationsvorrichtung erlaubt eine gleichmäßige und präzise Medikamentendosierung unabhängig vom Koordinationsvermögen des Patienten. Durch unterschiedliche Volumina des Behältnisses ist der gewünschte Depositionsort in der Lunge und auch die gewünschte Aerosolmenge vorwählbar. Bei zumindest teilweise durchsichtiger Ausgestaltung des Gehäuses kann das inhalierte Volumen visuell kontrolliert werden, da der Patient sieht, wie sich das Behältnis zusammenfaltet. Die erfindungsgemäße Inhalationsvorrichtung erlaubt eine einfache Handhabung und zugleich hohe Effektivität. Durch das Einbringen des Wirkstoffes in das Behältnis vor der Inhalation wird die Aerosolausgabe aus dem Spender auf die notwendige Menge beschränkt, was einen übermäßigen Verbrauch verhindert. Die genaue und effiziente Dosierung wiederum führt zu niedrigen Kosten der Behandlung beispielsweise mit Vitamin A. Ein weiterer Vorteil der Erfindung ist, daß die Verwendung eines Treibmittels, beispielsweise für die Verabreichung von Vitamin A, nicht zwingend erforderlich ist.

Der Begriff "geeignete Arzneistoffe" wie hier verwendet schließt Wirkstoffe, Medikamente, Verbindungen, Zusammensetzungen oder Mischungen von Stoffen ein, die einen pharmakologischen, - oft vorteilhaften - Effekt erzielen. Dies schließt Speisen, Nahrungsergänzungsstoffe, Nährstoffe, Medikamente, Impfstoffe, Vitamine und weitere nützliche Wirkstoffe mit ein. Die Ausdrücke, wie sie hier benutzt werden, schließen weiterhin alle physiologisch oder pharmakologisch aktiven Substanzen ein, die einen lokalen oder systemischen Effekt in einem Patienten bewirken. Der aktive Wirkstoff, der in Aerosolform zugeführt werden kann, schließt Antikörper, antivirale Wirkstoffe, Antiepileptika, Analgetika, entzündungshemmende Wirkstoffe und Bronchodilatatoren ein und kann eine organische oder anorganische Verbindung sein, die ohne Beschränkungen auch Medikamente einschließt, die auf das periphäre Nevensystem, adrenerge Rezeptoren, cholinerge Rezeptoren, Skelettmuskulatur, kardiovaskuläres System, glatte Muskulatur, Blutkreislaufsystem, neuronale Verbindungen, endokrines- und Hormonsystem, Immunsystem, reproduktives System, Skelettsystem, Nahrungszufuhr- und exkretorisches System, Histaminkaskade oder zentrales Nervensystem wirken. Geeignete Wirkstoffe können z.B. aus Polysacchariden, Steroiden, Hypnotika und Sedativa, antriebssteigernden Mitteln, Beruhigungsmitteln, krampflösenden und muskelentspannenden Mitteln, Antiparkinson-Substanzen, Analgetica, Entzündungshemmern, antimikrobiellen Wirkstoffen, Antimalariamitteln, Hormonen inklusive empfängnisverhütenden Mitteln, Symphaticomimetica, Polypeptiden und Proteinen, die physiologische Effekte hervorrufen, Diuretica, Fettstoffwechsel regulierenden Substanzen, antiandrogenen Wirkstoffen, gegen Parasiten gerichtete Mitteln, neoplastisch und antineoplastisch wirkenden Stoffen, Antidiabetika, Nahrungs- und Nahrungsergänzungsstoffen, wachstumsfördernden Stoffen, Fetten, stuhlregulierenden Stoffen, Elektrolyten, Impfstoffen und Diagnostika bestehen.

Die Erfindung ist besonders geeignet zur inhalativen Applikation von verschiedenen Wirkstoffen (ist aber nicht darauf beschränkt) wie:
Insulin, Calcitonin, Erythropoietin (EPO), Faktor VIII, Faktor IX, Cyclosporin, Granulozyte Colony Stimulating Factor (GCSF), Alpha-1 Proteinase Inhibitor, Elcatonin, Granulocyte Makrophage Colony Stimulating Factor (GMCSF), Wachstumshormone, menschliches Wachstumshormon (HGH), Growth hormon releasing hormone (GHRH), Heparin, niedermolekulares Heparin (LMWH), Interferon alpha, Interferon beta, Interferon gamma, Interleukin-2, Luteinizing hormone releasing hormone (LHRH), Somatostatin, Somatostatin-Anlaloge einsschließlich Octreotide, Vasopressinanaloge, Follikel stimulierendes Hormon (FSH), Insulin like growth factor, Insulintropin, Interleukin-I Rezeptorantagonist, Interleukin-3, Interleukin-4, Interleukin-6, Macrophage colony stimulating Factor (M-CSF), Nerven-Wachstumsfaktor, Parathyroidhormon (PTH), Thymosin alpha 1, IIb/IIIa Inhibitor, Alpha-1 Antitrypsin, Antikörper gegen respiratorisch syncytischen Virus, Cystic fibrosis transmembrane regulator gene (CFTR), Desoxyribonuclease (Dnase), Bactericide, permeability increasing protein (BPI), anti-CMV Anikörper, Interleukin-1 Rezeptor, Retinol, Retinyl-Ester, Tocopherole und deren Ester, Tocotrienole und deren Ester, Carotinoide insbesondere Beta-Carotin und andere natürliche und synthetische Antioxidantien, Retinol-Säuren, Pentamidine, Albuterolsulfat, Metaproterenolsulfat, Beclomethasondiprepionat, Triamcinolonacetamid, Budesonidacetonide, Ipratropiumbromid, Flunisolide, Fluticasone, Cromolynsodium, Ergotamintartrat und die Analogen, Agonisten und Antagonisten der oben genannten Stoffe. Aktive Wirkstoffe können weiterhin Nukleinsäuren in Form von reinen Nukleinsäuremolekülen, viralen Vektoren, assoziierten viralen Partikeln, Nukleinsäuren, die mit Lipiden oder einem Lipide beinhaltenden Material assoziiert oder darin enthalten sind, Plasmid-DNA oder -RNA oder andere Konstrukte aus Nukleinsäuren sein, die geeignet sind für die Zell-Transfection oder -Transformation, besonders bei Zellen der alveolären Region der Lunge. Der aktive Wirkstoff kann in verschiedenen Formen vorliegen, wie lösliche oder unlösliche geladene oder ungeladene Moleküle, Komponenten molekularer Komplexe oder pharmakologisch akzeptierte Hilfsstoffe. Der aktive Wirkstoff kann bestehen aus natürlich vorkommenden Molekülen, oder deren rekombinanter Produktion, oder die Moleküle können Analoge der natürlich vorkommenden oder rekombinant erzeugten aktiven Wirkstoffe sein, bei denen eine oder mehrere Aminosäuren addiert oder entfernt wurden. Weiterhin kann der aktive Wirkstoff abgeschwächten Lebendimpfstoff oder abgetötete Viren für den Impfstoffgebrauch enthalten. Im Falle des Wirkstoffes Insulin sind natürlich extrahiertes menschliches Insulin, rekombinantes menschliches Insulin, aus Rindern und/oder Schweinen extrahiertes Insulin, rekombinantes Schweine-oder Rinderinsulin und Mischungen der oben genannten Insuline eingeschlossen. Das Insulin kann in reiner, also in substanziell gereinigter Form vorliegen, kann aber auch Auszüge wie kommerziell üblich enthalten. Im Ausdruck "Insulin" sind auch Insulin-Analoge enthalten, bei denen eine oder mehrere der Aminosäuren des natürlich vorkommenden oder rekombinanten Insulins addiert oder entfernt wurden. Besonders ist die erfindungsgemäße Inhalationsvorrichtung für die Verabreichung von Vitamin A bzw. von Vitamin A-Ester und Retinsäure bzw. Retinsäure-Ester geeignet, auch in Kombination mit natürlichen und synthetischen Antioxidantien.

Im folgenden wird die Erfindung unter Bezugnahme auf die beigefügten Zeichnungen näher erläutert. Es zeigen:
- Fig. 1: einen Längsschnitt einer Inhalationsvorrichtung;
- Fig. 2: einen Querschnitt eines Beispiels einer Steuereinrichtung;
- Fig. 3: eine skizzenhaft perspektivische Darstellung des Behältnisses der Inhalationsvorrichtung;
- Fig. 4: eine Draufsicht einer weiteren Steuereinrichtung gemäß der EP-A-1 038 544;
- Fig. 5: einen Querschnitt der Steuereinrichtung gemäß Fig. 4;
- Fig. 6a,b: Schnittansichten zweier altemativer Ausführungsformen des Behältnisses;
- Fig. 7: eine Steuereinrichtung mit vier gleich langen Stegen;
- Fig. 8: die erfindungsgemäße Steuereinrichtung ;
- Fig. 9: eine Ausführungsform der flexiblen Matte;
- Fig. 10: eine alternative Ausführungsform der flexiblen Matte; und
- Fig. 11a,b: eine Draufsicht bzw. eine Schnittansicht einer Scheibe mit Stegen.

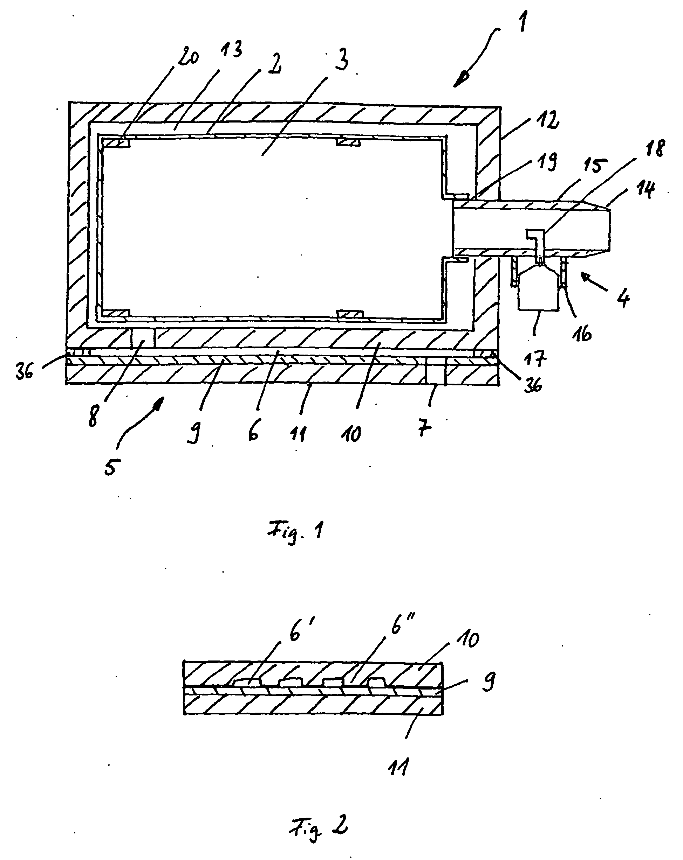

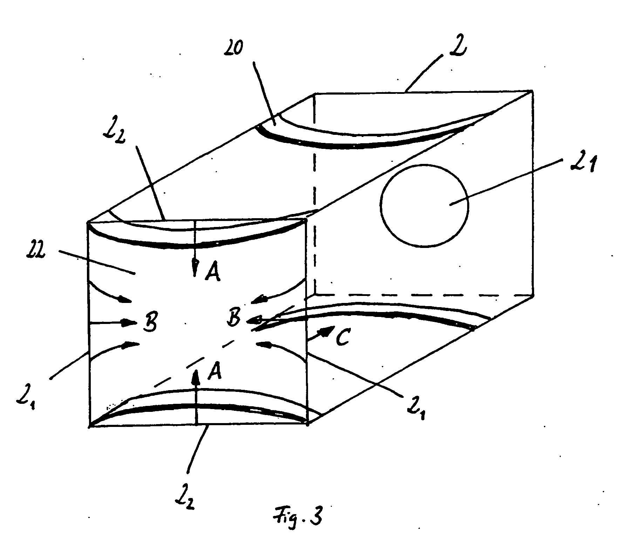

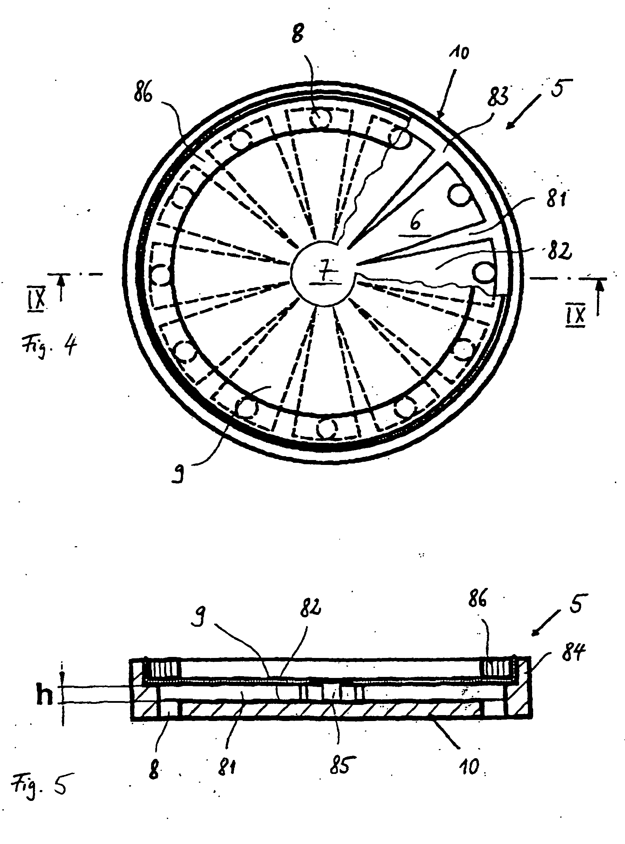

Die in Fig. 1 dargestellte Inhalationsvorrichtung 1 weist ein Gehäuse 12 auf, in dessen Innerem 13 ein Behältnis 2 (beispielsweise ein Ballon oder Beutel) für das Aerosolvolumen angeordnet ist. Ausgehend von einem Mundstück 14 erstreckt sich ein Kanal 15 in das Innere 13 des Gehäuses 12. An diesem Ende weist der Kanal 15 die Form eines Kragens 19 auf, auf den das Behältnis 2 aufgesteckt werden kann. Das Behältnis 2 ist vorzugsweise ein Einwegbehältnis. Das Gehäuse 12 ist vorzugsweise zumindest teilweise aus durchsichtigem Material.

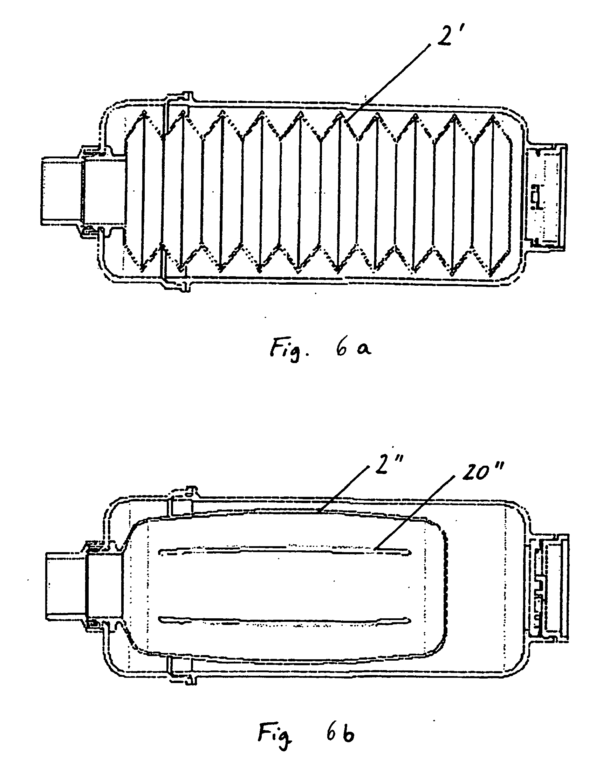

Am Kanal 15 ist ein Kragen 16 ausgebildet, der zusammen mit der Düse 18 eine Anordnung 4 bildet, über die Aerosol (beispielsweise Vitamin A) aus einer Kartusche 17 in das Innere des Behältnisses ausgegeben werden kann. Vorzugsweise ist die Düse 18 ein Vernebler, der in das Innere des Behältnisses 2 weist. Das Behältnis ist selbstexpandierend, so daß ein Vernebel des Aerosols eine Expansion des Behältnisses bewirkt. Wie in Fig. 6a gezeigt ist, ist das Behältnis vorzugsweise ein Faltenbalg 2'. Fig. 6b zeigt die Ausführungsform mit Beutel 2", der Verstärkungsrippen 20" aufweist, die ein ordnungsgemäßes Entfalten gewährleisten.

In der in Fig. 1 gezeigten Inhalationsvorrichtung stellt eine Wand 10 des Gehäuses 12 zugleich eine Begrenzungswand des Strömungskanals 6 dar. Dieser Strömungskanal ist weiterhin durch eine längliche, großflächige, flexible Silikonwand 9 beschränkt, die parallel zu der Wand 10 angeordnet ist. An den beiden Enden des Strömungskanals ist eine Einlaßöffnung 7 und eine Auslaßöffnung 8 vorgesehen. Sobald durch die Inhalation im Gehäuseinneren ein Unterdruck entsteht, wird über die Einlaßöffnung 7 Luft in das Innere des Strömungskanals 6 gesaugt und strömt durch diesen und über die Auslaßöffnung 8 in das Innere 13 des Gehäuses 12. Dabei bewirkt der anliegende Unterdruck, daß sich die Silikonmatte verbiegt bzw. nach innen wölbt und den Querschnitt des Strömungskanals reduziert. Der anliegende Unterdruck nimmt in Längsrichtung des Strömungskanals von Auslaßöffnung 8 hin zur Einlaßöffnung 7 ab. Diese Silikonmatte 9 ist von einer Abdeckung 11 abgedeckt. In dieser ist die Auslaßöffnung 7 vorgesehen. In einem bevorzugten Beispiel weist der Strömungskanal 6 mehrere Vertiefungen 6' auf, die durch entsprechende Längsstege 6" voneinander beabstandet sind. Dies ist in Fig. 2 gezeigt.

Das Behältnis 2 weist vorzugsweise mindestens eine Verstrebung 20 auf. Das in den Figuren 1 und 3 gezeigte Behältnis 2 zeigt vier Verstrebungen 20. Diese sind vorzugsweise bogenförmig ausgebildet und weisen in das Innere des Behältnisses 2. Die Verstrebungen 20 sind voneinander beabstandet an gegenüberliegenden Wänden des Behältnisses 2 angebracht, so daß sich jeweils zwei Verstrebungen gegenüberliegen. Vorzugsweise verlaufen die Verstrebungen 20 senkrecht zum Kanal 15. Diese Verstrebungen unterstützen ein definiertes Zusammenfalten des Behältnisses 2 bei der Inhalation. Während der Inhalation bewegen sich die in Fig. 3 gezeigten horizontalen Wände 2₂ des Behältnisses 2 in Pfeilrichtung A aufeinander zu, während die vertikalen Wände 2₁ sich in Pfeilrichtung B nach innen falten. Die der Öffnung 21 gegenüberliegende Wand 22 des Behältnisses 2 bewegt sich in Pfeilrichtung C auf die Öffnung 21 zu. Dieses definierte Zusammenfalten des Behältnisses 2 erlaubt ein nahezu vollständiges Inhalieren des Aerosolvolumens, da sich das Behältnis 2 an der Öffnung 21 definiert zusammenzieht und im wesentlichen keine Toträume entstehen.

In den Figuren 4 und 5 ist eine weitere Steuereinrichtung dargestellt. Hier besteht die Steuereinrichtung aus einer scheibenförmigen Wand 10 mit einer scheibenförmigen Ausnehmung 85. In der Ausnehmung 85 sind Stege 81 der Höhe h ausgebildet, die Strömungskanäle 6 bilden. Die Strömungskanäle 6 verbinden eine zentrale Einlaßöffnung 7 mit den ringförmig angeordneten Auslassöffnungen 8. Die Ausnehmung der scheibenförmigen Wand 10 weist einen abgestuften Bereich 84 auf, in dem eine flexible Matte 9 zu liegen kommt, die randseitig mittels eines Befestigungsringes 86 an der Wand 10 festgeklemmt ist. Die Einlaßöffnung 7 ist in Form einer mittigen Öffnung in der Matte 9 vorgesehen.

Bei der dargestellten Steuereinrichtung fließt Luft über die zentrale Einlaßöffnung 7 über die sternförmig angeordneten Strömungskanäle 6 hin zu den Auslassöffnungen 8. Durch den dabei entstehenden Unterdruck wird die flexible Matte 9, wie bei der in Fig. 2 dargestellten Ausführungsform, in die Strömungskanäle hinein gewölbt und verursacht dadurch eine Verengung des Querschnitts des Strömungskanals. Alternativ kann die Luftströmung in umgekehrter Richtung erfolgen.

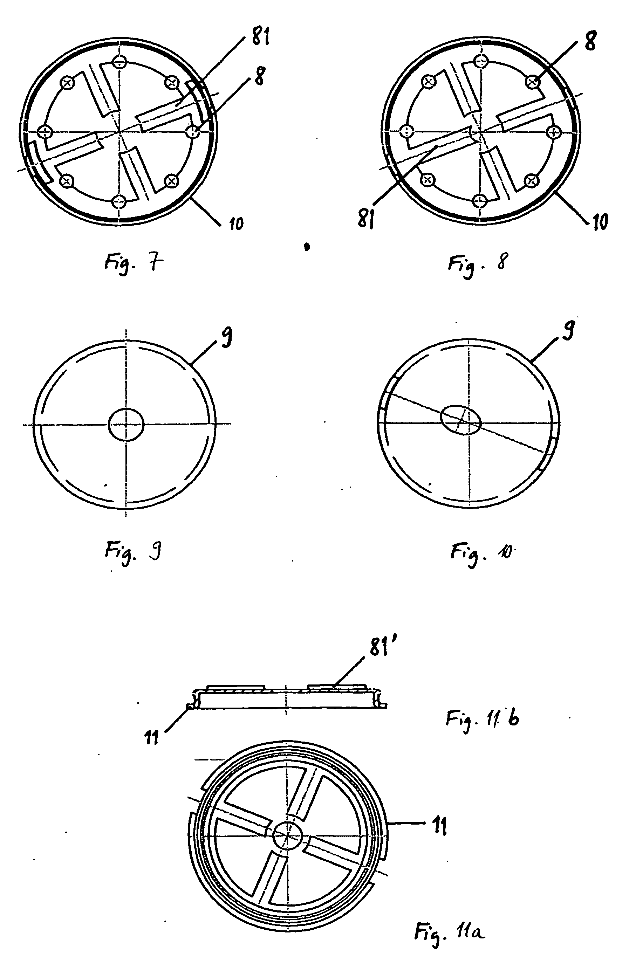

Fig. 7 zeigt eine gegenüber der Steuereinrichtung von Fig. 4 leicht modifizierte Steuereinrichtung. Diese Steuereinrichtung weist vier Stege 81 im Abstand von etwa 90° auf, zwischen denen je zwei Auslassöffnungen 8 vorgesehen sind. Die Auslassöffnungen sind über die zwischen den Stegen ausgebildeten Strömungskanäle mit der Einlassöffnung 7 verbunden. Die Einlassöffnung ist dabei in der Matte 9 entweder kreisförmig ausgebildet und zentriert angeordnet (Fig. 9), oder aber in Form eines Ovals und/oder außermittig angeordnet (Fig. 10). In der erfindungsgemäßen Ausführungsform (Fig. 8) ist ein Steg länger als die anderen (dies ist in der Zeichnung der mit dem Bezugszeichen 81 versehene Steg). Diese Modifikation verhindert ein vollständiges Verschließen des Strömungskanals durch die Matte 9, da diese somit nicht vollständig im Zentrum auf der scheibenförmigen Wand 10 aufliegen kann. Auch die ovale Öffnung in der Matte 9 (vgl. Fig. 10) verhindert ein vollständiges Verschließen.

Weiter bevorzugt sind die Stege nicht nur in der scheibenförmigen Wand 10 vorgesehen, sondern ebenfalls in einer auf der anderen Seite der Matte 9 angeordneten Wand oder kreisförmigen Scheibe 11 (vgl. Fig. 11a,b), die die Matte von der anderen Seite trägt. Dies sind die in den Figuren gezeigten Stege 81'. Diese beidseitige Ausbildung von Stegen vereinfacht die Verwendung der Inhalationsvorrichtung in jeder Lage (waagrecht, senkrecht, geneigt), weil die Matte 9 beidseitig durch die Stege in ihrer Lage gehalten wird. Die mittige Öffnung der gegenüberliegenden Scheibe 11 ist dabei mindestens so groß wie die Öffnung in der Matte.

Fig. 6a zeigt eine zylinderförmige Inhalationsvorrichtung, die als Behältnis einen Faltenbalg 2' verwendet (In Fig. 60b ist ein Beutel 2" mit Verstärkungsrippen 20" gezeigt). An einem Ende (in der Zeichnung links) ist die Öffnung vorgesehen, über die Aerosol aus dem Behältnis dem Patienten zugeführt wird. Am gegenüberliegenden Ende (in der Zeichnung links) ist eine Steuereinrichtung gemäß Figuren 7 oder 8 vorgesehen.

Die Inhalationsvorrichtung weist neben dem selbstexpandierbaren Behältnis 2 für ein vorbestimmtes Aerosolvolumen und der Einrichtung 4 zum Einbringen von Aerosol, z. B. Retinol, Retinyl-Ester, Retinsäure bzw. Retinsäure-Ester, aus einem Aerosolspender 17 in das Behältnis 2 bevorzugt eine Steuereinrichtungen zum Steuern des Inhalationsflusses auf.

## Patentansprüche

1. Inhalationsvorrichtung, mit:
einem selbstexpandierbaren Behältnis (2) für ein vorbestimmtes Aerosolvolumen (3);
einer Einrichtung (4) zum Einbringen von Aerosol aus einem Aerosolspender (17) in das Behältnis (2); und
einer Steuereinrichtung (5) zum Steuern des Inhalationsflusses,
**dadurch gekennzeichnet, daß** die Steuereinrichtung (5) den Inhalationsfluss während der gesamten Inhalation des Aerosols im wesentlichen konstant hält, wobei die Steuereinrichtung (5) vier zwischen einer zentralen Einlaßöffnung (7) und zu der Einlaßöffnung (7) radial beabstandeten Auslaßöffnungen (8) radial verlaufende Strömungskanäle (6) aufweist, wobei die vier radial verlaufenden Strömungskanäle (6) durch vier im Abstand von etwa 90° sternförmig angeordnete Stege (81) gebildet sind, die sich von einer im wesentlichen starren Wand (10) zu einer im wesentlichen flexiblen Wand (9) erstrecken, wobei ein Steg länger ist als die anderen.

2. Inhalationsvorrichtung nach Anspruch 1, wobei die Strömungskanäle (6) durch eine sich entlang der Strömungskanäle (6) erstreckende großflächige flexible Wand (9) begrenzt sind.

3. Inhalationsvorrichtung nach Anspruch 2, wobei die flexible Wand (9) den Querschnitt der Strömungskanäle (6) durch den bei einer Inhalation des Aerosols entstehenden Unterdruck reduziert.

4. Inhalationsvorrichtung nach Anspruch 2 oder 3, wobei die Strömungskanäle (6) ferner durch eine erste, im wesentlichen starre Wand (10) begrenzt werden.

5. Inhalationsvorrichtung nach einem der Ansprüche 2 bis 4, wobei die flexible Wand (9) zwischen der mindestens einen starren Wand (10) und einer Abdeckung (11) angeordnet ist.

6. Inhalationsvorrichtung nach einem der Ansprüche 4 oder 5, wobei die Auslaßöffnungen (8) in der einen starren Wand (10) angeordnet sind.

7. Inhalationsvorrichtung nach Anspruch 5 oder 6, wobei die Einlaßöffnung (7) in der Abdeckung (11) angeordnet ist.

8. Inhalationsvorrichtung nach einem der Ansprüche 1 bis 7, ferner mit einem Gehäuse für das Behältnis.

9. Inhalationsvorrichtung nach Anspruch 8, wobei die erste, im wesentlichen starre Wand (10) mit dem Gehäuse (12) einstückig ausgebildet ist.

10. Inhalationsvorrichtung nach Anspruch 8 oder 9, wobei die Auslaßöffnungen (8) die Strömungskanäle (6) mit dem Gehäuseinneren (13) verbinden.

11. Inhalationsvorrichtung nach einem der Ansprüche 2 bis 10, wobei die Einlaßöffnung (7) und die Auslaßöffnungen (8) senkrecht zu den Strömungskanälen (6) angeordnet sind.

12. Inhalationsvorrichtung nach einem der Ansprüche 2 bis 11, wobei die flexible Wand (9) aus einem bioverträglichen Material besteht.

13. Inhalationsvorrichtung nach einem der Ansprüche 1 bis 12, wobei ein Kanal (15) ein Mundstück (14) mit dem Gehäuseinneren verbindet und sich in das Gehäuseinnere (13) erstreckt und an dem dortigen Ende einen Kragen (19) aufweist.

14. Inhalationsvorrichtung nach Anspruch 13, wobei das Behältnis (2) an dem Kragen (19) anbringbar ist.

15. Inhalationsvorrichtung nach Anspruch 13 oder 14, wobei die Einrichtung (4) zum Einbringen von Aerosol aus einem Aerosolspender (17) an dem Kanal (15) angeordnet ist.

16. Inhalationsvorrichtung nach einem der Ansprüche 1 bis 15, wobei die Einbringungseinrichtung (4) eine Halterung (16) für eine Kartusche (17) aufweist.

17. Inhalationsvorrichtung nach einem der Ansprüche 1 bis 16, wobei die Düse (18) ein Vernebler ist.

18. Inhalationsvorrichtung nach einem der Ansprüche 1 bis 17, wobei das Behältnis (2) ein Beutel, ein Ballon oder ein Faltenbalg ist, vorzugsweise aus einem biokompatiblen Material.

19. Inhalationsvorrichtung nach einem der Ansprüche 1 bis 18, wobei das Aerosol Vitamin A aufweist.

## Claims

1. An inhalation device comprising:
a self-expandable container (2) for a predetermined aerosol volume (3);
a means (4) for introducing aerosol from an aerosol dispenser (17) into the container (2); and
a control means (5) for controlling the inhalation flow,
**characterized in that** the control means (5) keeps the inhalation flow essentially constant during the entire inhalation period,
wherein the control means (5) comprises four flow channels (6) radially extending between a central inlet opening (7) and outlet openings (8) radially spaced apart from the inlet opening (7), wherein the four radially extending flow channels (6) are formed by four ribs (81) spaced approximately 90° apart in form of a star and extend from an essentially stiff wall (10) to an essentially flexible wall (9), wherein one web is longer that the others.

2. The inhalation device according to claim 1, wherein the flow channels (6) are delimited by a flexible large surface wall (9) extending along the flow channels (6).

3. The inhalation device according to claim 2, wherein the flexible wall (9) reduces the cross section of the flow channels (6) via the negative pressure produced by an inhalation of the aerosol.

4. The inhalation device according to claim 2 or 3, wherein the flow channels (6) are additionally delimited by a first essentially stiff wall (10).

5. The inhalation device according to any of claims 2 to 4, wherein the flexible wall (9) is arranged between the at least one stiff wall (10) and a cover (11).

6. The inhalation device according to claim 4 or 5, wherein the outlet openings (8) are arranged in the one stiff wall (10).

7. The inhalation device according to claim 5 or 6, wherein the inlet opening (7) is arranged in the cover (11).

8. The inhalation device according to any of claims 1 to 7 further comprising a housing for the container.

9. The inhalation device according to claim 8, wherein the first essentially stiff wall (10) is integrally formed with the housing (12).

10. The inhalation device according to claim 8 or 9, wherein the outlet openings (8) connect the flow channels (6) to the interior (13) of the housing.

11. The inhalation device according to any of claims 2 to 10, wherein the inlet opening (7) and the outlet openings (8) are arranged perpendicularly to the flow channels (6).

12. The inhalation device according to any of claims 2 to 11, wherein the flexible wall (9) is made of a bio-compatible material.

13. The inhalation device according to any of claims 1 to 12, wherein a channel (15) connects a mouthpiece (14) to the interior of the housing and extends into the interior (13) of the housing and has a collar (19) at its end there.

14. The inhalation device according to claim 13, wherein the container (2) can be attached to the collar (19).

15. The inhalation device according to claim 13 or 14, wherein the means (4) for introducing aerosol from an aerosol dispenser (17) is arranged at the channel (15).

16. The inhalation device according to any of claims 1 to 15, wherein the introduction means (4) has a holder (16) for a cartridge (17).

17. The inhalation device according to any of claims 1 to 16, wherein the nozzle (18) is a nebulizer.

18. The inhalation device according to any of claims 1 to 17, wherein the container (2) is a bag, a balloon, or an expansion bellows, preferably made of a bio-compatible material.

19. The inhalation device according to any of claims 1 to 18, wherein the aerosol contains vitamin A.

## Revendications

1. Inhalateur comprenant :
un récipient (2) auto-expansible pour un volume d'aérosol (3) prédéterminé ;
un dispositif (4) pour introduire l'aérosol depuis un distributeur d'aérosol (17) dans le récipient (2) ; et
un dispositif de commande (5) pour commander le flux d'inhalation,
**caractérisé en ce que** le dispositif de commande (5) maintient le flux inhalé essentiellement constant pendant toute l'inhalation de l'aérosol, le dispositif de commande (5) comportant quatre canaux d'écoulement (6) passant radialement entre une ouverture d'entrée centrale (7) et des ouvertures de sortie (8) à distance radiale de l'ouverture d'entrée (7), que les quatre canaux d'écoulement (6) passant radialement étant formés par quatre nervures (81) disposées en forme d'étoile à une distance de 90° environ, lesquelles s'étendent depuis une paroi (10) essentiellement rigide vers une paroi (9) essentiellement flexible, une nervure étant plus longue que les autres.

2. Inhalateur selon la revendication 1, dans lequel les canaux d'écoulement (6) sont délimités par une paroi (9) flexible à grande surface s'étendant le long des canaux d'écoulement (6).

3. Inhalateur selon la revendication 2, dans lequel la paroi flexible (9) réduit la section transversale des canaux d'écoulement (6) par la dépression générée lors d'une inhalation de l'aérosol.

4. Inhalateur selon la revendication 2 ou 3, dans lequel les canaux d'écoulement (6) sont en outre limités par une première paroi (10), essentiellement rigide.

5. Inhalateur selon l'une quelconque des revendications 2 à 4, dans lequel la paroi flexible (9) est disposée entre l'au moins une paroi rigide (10) et un recouvrement (11).

6. Inhalateur selon l'une quelconque des revendications 4 ou 5, dans lequel les ouvertures de sortie (8) sont disposées dans la paroi rigide (10).

7. Inhalateur selon la revendication 5 ou 6, dans lequel l'ouverture d'entrée (7) est placée dans le recouvrement (11).

8. Inhalateur selon l'une quelconque des revendications 1 à 7, avec en outre un boîtier pour le récipient.

9. Inhalateur selon la revendication 8, dans lequel la première paroi (10) essentiellement rigide est formée d'un tenant avec le boîtier (12).

10. Inhalateur selon la revendication 8 ou 9, dans lequel les ouvertures de sortie (8) relient les canaux d'écoulement (6) à l'espace intérieur du boîtier (13).

11. Inhalateur selon l'une quelconque des revendications 2 à 10, dans lequel l'ouverture d'entrée (7) et les ouvertures de sortie (8) sont perpendiculaires aux canaux d'écoulement (6).

12. Inhalateur selon l'une quelconque des revendications 2 à 11, dans lequel la paroi flexible (9) se compose d'un matériau biocompatible.

13. Inhalateur selon l'une quelconque des revendications 1 à 12, dans lequel un canal (15) relie un bec (14) à l'espace intérieur du boîtier et s'étend dans l'espace intérieur du boîtier (13) et comporte un collet (19) à l'extrémité qui s'y trouve.

14. Inhalateur selon la revendication 13, dans lequel le récipient (2) peut être mis sur le collet (19).

15. Inhalateur selon la revendication 13 ou 14, dans lequel le dispositif (4) destiné à l'introduction de l'aérosol depuis un distributeur d'aérosol (17) est disposé au niveau du canal (15).

16. Inhalateur selon l'une quelconque des revendications 1 à 15, dans lequel le dispositif d'introduction (4) comporte un support (16) pour une cartouche (17).

17. Inhalateur selon l'une quelconque des revendications 1 à 16, dans lequel la buse (18) est un nébuliseur.

18. Inhalateur selon l'une quelconque des revendications 1 à 17, dans lequel le récipient (2) est un sachet, un ballon ou un soufflet, fabriqué de préférence à partir d'un matériau biocompatible.

19. Inhalateur selon l'une quelconque des revendications 1 à 18, dans lequel l'aérosol comporte de la vitamine A.
